# EUROPÄISCHE PATENTSCHRIFT

(11) **EP 0 697 440 B1**
(45) Veröffentlichungstag und Bekanntmachung des Hinweises auf die Patenterteilung: **14.11.2001**
(21) Anmeldenummer: 95810483.8
(22) Anmeldetag: 25.07.1995
(51) Int. Cl.: C08L 63/00, C08L 67/00, C08L 33/06, C08G 59/68, C08G 59/12

(54) **Thermisch härtbare Zusammensetzung, insbesondere für die Verwendung als Autolack**
Heat-hardenable composition, especially suited for use as automotive paints
Composition thermodurcissable, et leur application notamment comme peintures pour véhicules

(30) Priorität: 02.08.1994 CH 241794
(43) Veröffentlichungstag der Anmeldung: 21.02.1996
(73) Patentinhaber: Vantico AG, 4057 Basel (CH)
(72) Erfinder: Frischinger, Isabelle, Dr., F-68640 Riespach (FR); Cotting, Jacques-Alain, Dr., CH-1729 Bonnefontaine (CH); François, Jacques, F-6330 Huningue (FR)

(56) Entgegenhaltungen:
- EP-A- 0 212 457
- EP-A- 0 330 887
- DE-A- 4 237 658

## Beschreibung

Die Erfindung bezieht sich auf Zusammensetzungen, die sich insbesondere als Autolacke eignen, und betrifft speziell härtbare Zusammensetzungen auf Basis von freie Carboxylgruppen enthaltenden Polymeren und Epoxidharzen. Die Erfindung betrifft ferner neue Glycidylester und Glycidylestergemische, die sich insbesondere für die genannten Zusammensetzungen eignen, und ein Verfahren zur Lackierung von Gegenständen unter Verwendung der Zusammensetzungen.

In der Praxis häufig benutzte Farb- und Klarlacksysteme basieren auf 2-Komponentenzusammensetzungen aus Polyolen, wie Polyesterpolyolen, Polyurethanpolyolen oder Polyolen auf Acrylatbasis, und Polyisocyanaten als Härter. Die Polyisocyanate sind allerdings schwierig zu handhaben, empfindlich gegen Feuchtigkeit und ermöglichen nur ein kurzes Potlife für die Lacke. Ausserdem ist eine besondere Vorsorge wegen der Toxizität der Isocyanate erforderlich.

Aus dem US-Patent US-A-3,954,712 sind härtbare Zusammensetzungen auf Basis von Polyepoxidverbindungen, Halbestern von Diolen und Polycarbonsäureanhydriden bekannt.

In der EP-A-0 212 457 sind darüberhinaus härtbare Zusammensetzungen auf Basis von freie Carboxylgruppen enthaltenden Polymeren und Epoxidharzen sowie deren Verwendung als Autodecklack beschrieben. Diese Zusammensetzungen können als Carboxylgruppen enthaltende Polymerkomponente unter anderem ein Gemisch aus einem Polymer auf Basis von Acrylat- und/oder Methacrylatmonomeren, das bevorzugt 0,54 bis 2,7 Äquivalente freie Carboxylgruppen pro Kilogramm Polymer und einen T_{g}-Wert unter 30 °C aufweist, und/oder einem carboxylterminierten Polyester, der etwa 0,2 bis 2,1 Äquivalente freie Carboxylgruppen pro Kilogramm Polyester und ebenfalls einen Tg-Wert unter 30 °C aufweist, enthalten. Als geeignete Epoxidharze führt die EP-A-0 212 457 neben den bevorzugten Epoxyacrylaten Glycidylether von aliphatischen sowie von aromatischen Polyhydroxyverbindungen mit vorzugsweise mehr als einer und bis zu drei Epoxidgruppen pro Molekül sowie difunktionelle cycloaliphatische Epoxidharze, d. h. Verbindungen mit 2 Epoxycycloalkylgruppen, an. Die beschriebenen härtbaren Zusammensetzungen weisen ein molares Verhältnis von freien Carboxylgruppen zu Epoxidgruppen von 0,3 bis 3 auf und können als weitere Bestandteile unter anderem geeignete Katalysatoren zur Beschleunigung der Reaktion von Epoxid- mit Carboxylatkomponenten sowie geeignete inerte Lösungsmittel enthalten.

Die Beschichtungen auf Basis der oben genannten Zusammensetzungen sind noch verbesserungsfähig, insbesondere im Hinblick auf ihre Härte, Flexibilität, ihre Widerstandskraft gegen organische Flüssigkeiten, z. B. gegen Benzin, und ihre Wetterbeständigkeit.

Gegenstand der vorliegenden Erfindung ist eine härtbare Zusammensetzung, die folgende Bestandteile enthält:
A) ein Gemisch aus
   A1) mindestens einem Polymer auf Basis von Acrylat- und/oder Methacrylatmonomeren, das durchschnittlich 0,1 bis 4,0 Äquivalente freie Carboxylgruppen pro Kilogramm Polymer aufweist, und
   A2) mindestens einem carboxylterminierten Polyester, der durchschnittlich 0,2 bis 6 Äquivalente freie Carboxylgruppen pro Kilogramm Polyester aufweist, wobei Bestandteil A1) 50 bis 90 Gewichtsprozent und Bestandteil A2) den Rest der Komponente A ausmachen;
B) einen oder mehrere Glycidylester cycloaliphatischer Carbonsäuren mit 2 oder mehr Epoxidgruppen pro Molekül oder ein Gemisch, das aus einem oder mehreren Glycidylestern cycloaliphatischer Carbonsäuren mit 2 oder mehr Epoxidgruppen pro Molekül und einem oder mehreren Glycidylestern aromatischer Carbonsäuren mit 2 oder mehr Epoxidgruppen pro Molekül besteht;
C) einen Katalysator zur Beschleunigung der thermischen Reaktion von Epoxid- und Carboxylgruppen und
D) ein inertes Lösungsmittel,
wobei das molare Verhältnis von freien Carboxylgruppen zu Epoxidgruppen in der Zusammensetzung 0,3 bis 3 beträgt und die Zusammensetzung ausser der Komponente B keine Bestandteile mit Epoxidgruppen aufweist.

Die erfindungsgemässen Zusammensetzungen sind sehr reaktiv und führen zu Beschichtungen besonderer Härte und Lösungsmittelbeständigkeit. Zudem zeigen die Beschichtungen unter anderem auch gute Wetterbeständigkeit, Elastizität und Schlagfestigkeit. Die Zusammensetzungen können weiterhin mit relativ hohem Feststoffgehalt, beispielsweise von 45 Gewichtsprozent und mehr, und somit entsprechend niedrigem Gehalt an Lösungsmitteln hergestellt werden. Auch bei derartig hohen Festkörpergehalten weisen die erfindungsgemässen Zusammensetzungen eine sehr niedrige Viskosität auf. So können zum Beispiel entsprechend hochfeststoffhaltige Lacke auf Basis der erfindungegemässen Zusammensetzungen hergestellt werden, deren Spritzviskosität einer Auslaufzeit von 18 Sekunden (DIN-4-Becher) entspricht.

Die für die erfindungsgemässen Zusammensetzungen Verwendung findenden Epoxidharze weisen zweckmässig ein Molekulargewicht von etwa 200 bis 2000 auf. Selbstverständlich können auch Gemische zweier oder mehrerer der angegebenen Epoxidharze Verwendung finden.

Unter Glycidylestern cycloaliphatischer bzw. aromatischer Carbonsäuren werden in dieser Anmeldung Polyglycidylester von Carbonsäuren verstanden, die mindestens zwei Carboxylgruppen aufweisen, die an Ringsysteme des Carbonsäuremoleküls gebunden sind. Diese Carbonsäuren können nur ein Ringsystem aufweisen, vorzugsweise von 5 bis 14 Ringkohlenstoffatomen, wie z. B. Polycarbonsäurederivate von Benzol oder Naphthalin. Sie können aber auch zwei oder mehrere derartige Ringsysteme in ihrem Molekül enthalten, die über geeignete Atomgruppen verknüpft sind, beispielsweise eine Gruppe der Formel -CH₂-, -C(CH₃)₂-, -O- oder 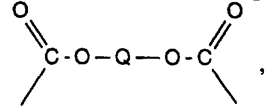 worin Q einen 2-wertigen organischen Rest mit 2 bis 15 Kohlenstoffatomen bedeutet.

Die erfindungsgemäss geeigneten Glycidylester umfassen z. B. Polyglycidylester cyclischer Polycarbonsäuren, wie von Dicarbonsäuren, beispielsweise Phthalsäure, Isophthalsäure, Terephthalsäure, 2,5-Dimethylphthalsäure, Naphtalin-2,6-dicarbonsäure, Naphtalin-1,8-dicarbonsäure, Naphtalin-2,3-dicarbonsäure, Diphenylether-4,4'-dicarbonsäure, Diphenyl-2,2'-dicarbonsäure, Tetrachlorphthalsäure, 2,5-Dichlorphthalsäure, o-, m- oder p-Phenylendiessigsäure, Tetrahydrophthalsäure, Methyltetrahydrophthalsäure, Hexahydrophthalsäure, Methylhexahydrophthalsäure, Endomethylen-hexahydrophthalsäure, Hexahydroterephthalsäure, Hexahydroisophthalsäure, Thiophen-2,5-dicarbonsäure, Furan-2,5-dicarbonsäure, Furan-3,4-dicarbonsäure, Pyrazin-3,4-dicarbonsäure, oder von höherfunktionellen Carbonsäuren, wie 1,2,3-Benzoltricarbonsäure (Hemimellitsäure), 1,2,4-Benzoltricarbonsäure (Trimellitsäure), 1,3,5-Benzoltricarbonsäure (Trimesinsäure), 1,2,3,4-Benzoltetracarbonsäure (Mellophansäure), 1,2,4,5-Benzoltetracarbonsäure (Pyromellitsäure), 1,2,3,5-Benzoltetracarbonsäure, Benzolpentacarbonsäure und Benzolhexacarbonsäure (Mellitsäure), Naphthalintetracarbonsäure, Perylentetracarbonsäure oder Tetracarbonsäuren der Formel 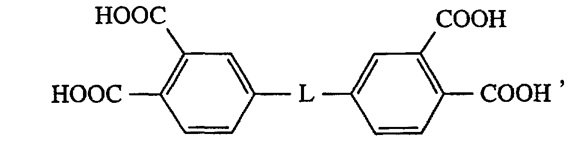 worin L für -CH₂-, -C(CH₃)₂- oder -O- steht, insbesondere Benzophenon-3,3',4,4'-tetracarbonsäure, sowie Derivate der genannten Säuren, die teilweise oder vollständig hydrierte Kohlenstoffringe aufweisen.

Die Polyglycidylester können nach bekannten Methoden oder analog zu diesen hergestellt werden, z. B. gemäss dem US-Patent US-A-3,859,314, der DE-A-31 26 411 oder der EP-A-506 617 hergestellt werden. Ganz besonders bevorzugte Glycidylester sind cis-Hexahydrophthalsäurediglycidylester sowie insbesondere die Triglycidylester von Trimellit- und Hexahydrotrimellitsäure.

Bevorzugte Zusammensetzungen enthalten ein Epoxidharz ausgewählt aus der Gruppe bestehend aus cis-Hexahydrophthalsäurediglycidylester und 1,2,4-Cyclohexantricarbonsäuretriglycidylester.

Besonders bevorzugt, sowohl wegen ihres guten Einflusses auf die Wetterbeständigkeit von Beschichtungen auf Basis erfindungsgemässer Zusammensetzungen als auch wegen ihrer vergleichsweise guten Reaktivität sind Cyclohexanpolycarbonsäureglycidylester mit mehr als zwei Glycidylestergruppen, wie die Triglycidylester von Hexahydrotrimesinsäure und insbesondere von Hexahydrotrimellitsäure. Cyclohexanpolycarbonsäureglycidylester mit mehr als 2 Glycidylestergruppen sind noch neu und bilden ebenfalls einen Gegenstand dieser Erfindung. Sie können, wie bekannte Glycidylester, beispielsweise durch Umsetzung der entsprechenden Cyclohexanpolycarbonsäure mit Epichlorhydrin hergestellt werden. Die genannten Cyclohexanpolycarbonsäureglycidylester können vorteilhaft auch im Gemisch mit Polyglycidylestern aromatischer Carbonsäuren als Bestandteil erfindungsgemässer Zusammensetzungen eingesetzt werden, wobei die Cyclohexanpolycarbonsäureglycidylester bevorzugt 30 bis 90 Gewichtsprozent des Gemisches, insbesondere 50 bis 90 Gewichtsprozent ausmachen.

Erfindungsgemässe Zusammensetzungen, die als Epoxidharz Glycidylester cyclischer Carbonsäuren enthalten, stellen eine bevorzugte Ausführungsform der Erfindung dar, ebenso wie diejenigen Zusammensetzungen mit Epoxidharzen, die 3 oder mehr Epoxidgruppen pro Molekül aufweisen.

Das Polymer auf Basis von Acrylat- und/oder Methacrylatmonomeren, das die Komponente A1) des Gemisches A der beanspruchten Zusammensetzungen bildet, ist ein Copolymer von einem oder mehreren Acrylsäure- und/oder Methacrylsäureestern, bevorzugt den entsprechenden Alkylestern mit 1 bis 8 Kohlenstoffatomen in der Alkylgruppe, mit Acrylsäure und/oder Methacrylsäure und gegebenenfalls weiteren ethylenisch ungesättigten Comonomeren und weist bevorzugt ein Molekulargewicht von 1000 bis 30000 auf. Seine Glasübergangstemperatur liegt zweckmässigerweise über 20 °C, bevorzugt über 30 °C. Beispiele für geeignete (Meth)acrylsäureestermonomere sind Ethylacrylat, Butylacrylat, 2-Ethylhexylacrylat, sowie insbesondere C₁-C₄-Alkylmethacrylate, wie Methylmethacrylat, Ethylmethacrylat oder Butylmethacrylat. (Meth)acrylatderivate, die Silangruppen enthalten können ebenfalls eingesetzt werden. Als ethylenisch ungesättigte Comonomere kommen beispielsweise Acryl- oder Methacrylnitrile sowie Vinylverbindungen in Frage. Bevorzugte Comonomere sind Vinylaromaten, insbesondere Styrol. Besonders bevorzugt wird als Komponente A1 ein Copolymer von Methacrylsäure, Methacrylsäure-C₁-C₄-alkylestern und Styrol eingesetzt. Die oben genannte Polymere können in bekannter Weise hergestellt werden, z. B. durch Polymerisation der in geeigneten organischen Lösungsmitteln, insbesondere in Toluol oder in Gemischen aus 1-Methoxy-2-propanol, 1-Methoxy-2-propylacetat und Methylisobutylketon (beispielsweise im Gewichtsverhältnis von 70/20/10), gelösten Monomeren in Gegenwart eines geeigneten Initiators, wie z. B. Dicumylperoxid, und eines Kettenübertragungsreagenzes, wie Thioglycolsäure.

Die carboxylterminierten Polyester der Komponente A2) weisen zweckmässig ein Molekulargewicht von 500 bis 5000, bevorzugt bis zu 2000, insbesondere bis zu 1000 auf. Ihre Glasübergangstemperatur liegt vorzugsweise unter 30 °C, besonders bevorzugt unter 0°C. Derartige Polyester sind bei Raumtemperatur fliessfähig.

Bevorzugte Polyester der vorgenannten Art sind Halbester aus einem Polyesterpolyol oder einem Polylactonpolyol, die jeweils mindestens 2 terminale Hydroxylgruppen aufweisen, und einer aliphatischen oder cycloaliphatischen 1,2-Dicarbonsäure. Diese Halbester weisen die nachstehende Molekularstruktur auf: 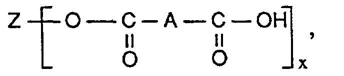 wobei Z den Rest eines Polyester- oder Polylactonpolyols der Formel Z(OH)ₓ ohne seine terminalen Hydroxylgruppen darstellt, A den Rest einer von ihren 1,2-ständigen Carboxylgruppen befreiten 1,2-Carbonsäure der Formel A(COOH)₂ symbolisiert, mit der oder mit derem esterbildenden Derivat das Polyol umgesetzt wurde, und x eine ganze Zahl von mindestens 2 ist. Die Obergrenze für x liegt bevorzugt bei 4. Ganz besonders bevorzugt ist für x der Wert 2 oder 3. Besonders gut geeignet sind Halbester auf Basis von Bernsteinsäure und insbesondere von cycloaliphatischen Carbonsäuren, besonders von Hexahydrophthalsäure oder Hexahydrotrimellitsäure, als Säurekomponente.

Als Polyesterpolyole für die Herstellung der Komponente A2) können z. B. Umsetzungsprodukte von polyfunktionellen Carbonsäuren, insbesondere cycloaliphatischen oder aliphatischen, wie beispielsweise Hexahydroisophthalsäure, Hexahydroterephthalsäure, Hexahydrophthalsäure, Bernsteinsäure, Glutarsäure, Adipinsäure, Pimelinsäure, Suberinsäure (Korksäure), Azelainsäure, Sebacinsäure, mit einem Überschuss an aliphatischen Polyolen eingesetzt werden, insbesondere Di- und Triolen, wie beispielsweise Ethylenglycol, Propylenglycol, 1,3-Butandiol, 1,4-Butandiol, Neopentandiol, Isopentylglycol, 1,6 Hexandiol, 1,7-Heptandiol, 1,8-Octandiol, Glycerin, Trimethylolethan, Trimethylolpropan oder Cyclohexandiol verwendet werden. Bevorzugt werden sowohl lineare aliphatische Dicarbonsäuren als auch lineare aliphatische Diole von jeweils 5 bis bis 8 Methylengruppen in der aliphatischen Kette. Polyester dieser Art sind auch kommerziell erhältlich, z. B. unter der Markenbezeichnung K-Flex® 188 und 148 von King Industries.

Zur Herstellung der Komponente A2 der erfindungsgemässen Zusammensetzungen können als Polyole Z(OH)ₓ günstig auch bestimmte Polylactonpolyole zum Einsatz kommen nämlich Verbindungen der Formel 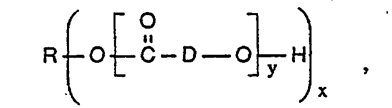 wobei R dem Rest eines x-wertigen als Initiator für die Lactonpolymerisierung verwendeten cyclischen oder acyclischen Polyols R(OH)ₓ ohne seine Hydroxylgruppen darstellt, x eine der oben für x schon genannte Bedeutung hat, y eins oder eine ganze Zahl grösser als 1, bevorzugt von 1 bis 20, insbesondere von 1 bis 10 bedeutet und D einer Alkylengruppe der Struktur 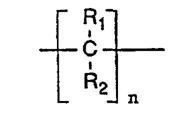 mit insgesamt maximal 12 Kohlenstoffatomen entspricht, wobei die Reste R₁ und R₂ unabhängig voneinander ein Wasserstoffatom oder einen C₁-C₃-Alkylrest darstellen und n eine ganze Zahl von 5 bis 8 ist. Polylactone der genannten Art sind bekannt und z. B. in der GB-A-2 160 534 beschrieben, deren Inhalt als Bestandteil dieser Beschreibung zu betrachten ist. Besonders bevorzugt ist n gleich 5 und sind R₁ und R₂ beide ein Wasserstoffatom. Derartige Polycaprolactone sind unter dem Handelsnamen CAPA® von Interox Chemicals Ltd. auch kommerziell erhältlich.

Die am meisten bevorzugte carboxylterminierte Polyester sind die Halbester aus hydroxylterminierten Polycaprolactonen und cis-Hexahydrophthalsäureanhydrid.

Die erfindungsgemässen Zusammensetzungen enthalten ausserdem einen Katalysator zur Beschleunigung der thermischen Reaktion von Epoxid- und Carboxylgruppen, der eine ausreichend schnelle Härtungsreaktion bei Temperaturen im Bereich von 60 bis 130° C ermöglicht. Im allgemeinen handelt es sich bei dem Katalysator um ein organisches Amin oder ein Derivat eines Amins, insbesondere um ein tertiäres Amin oder eine stickstoffhaltige heterocyclische Verbindung. Bevorzugte Katalysatoren sind N-Benzyldimethylamin und 1,8-Diazabicyclo[5,4,0]-7-undecen. Der Katalysator oder ein Katalysatorgemisch wird zweckmässig in einer Menge von etwa 0,5 bis 10, insbesondere von 1 bis 5 Gewichtsprozent zugesetzt

Beispiele für geeignete inerte Lösungsmittel sind Xylole, Butylacetat, Isobutanol, 1-Methoxy-2-propanol, 1-Methoxy-2-propylacetat und Methylisobutylketon (MIBK).

Besonders bevorzugt ist ein Lösungsmittelgemisch aus 1-Methoxy-2-propanol, 1-Methoxy-2-propylacetat und MIBK, bevorzugt im Gewichtsverhältnis von etwa 70 zu 20 zu 10. Die erfindungsgemässen Zusammensetzungen können mit relativ wenig inertem Lösungsmittel hergestellt werden und weisen daher bevorzugt einen hohen Festkörpergehalt, beispielsweise im Bereich von 40 bis 60 Gewichtsprozent für eine Spritzviskosität von 18 Sekunden Auslaufszeit DIN-4-Becher auf.

Die erfindungsgemässen Zusammensetzungen können ausser den genannten noch weitere Bestandteile enthalten, die für Lackfarben üblich sind, z. B. Pigmente, Aufheller, Lichtschutzmittel, Antioxidationsmittel, Verlaufsmittel, Haftmittel, Thixotropiermittel u. s. w..

Die erfindungsgemässen Zusammensetzungen können beispielsweise als Überzugsmittel, Giessharz, Imprägnierharz, Laminierharz, Klebstoff oder Dichtmaterial Anwendung finden. Besonders bevorzugt ist die Verwendung der erfindungsgemässen Zusammensetzungen als Automobillack, wobei sie beispielsweise als Grundierlack (Primer) oder, gefüllt mit Pigmenten und/oder Farbstoffen, als farbgebender Basislack sowie unpigmentiert als klarer Decklack verwendet werden können. Erfindungsgemässe Zusammensetzungen sind besonders gut für die Erstlackierung der Autos beim Hersteller geeignet (Original Equipment Manufacturing), wobei bevorzugt Einbrenntemperaturen von etwa 60 bis 180 °C angewandt werden. Besonders bei Verwendung als Decklack erweist es sich als vorteilhaft, dass die erfindungsgemässen Zusammensetzungen auch bei höheren Einbrenntemperaturen, beispielsweise von 130°C und höher, keinerlei Vergilbungstendenz erkennen lassen.

Die Erfindung hat daher auch ein Verfahren zur Lackierung von Gegenständen, insbesondere von Automobilteilen, zum Gegenstand, bei dem der Gegenstand mit einer erfindungsgemässen Zusammensetzung beschichtet wird und die Beschichtung getrocknet und bei einer Temperatur von 60 bis 180 °C, vorzugsweise von 80 bis 130 °C, gehärtet wird.

Falls in den Beispielen nicht anders angegeben, sind Prozentangaben als Gewichtsprozente zu verstehen. Zu den Beispielen gehörende identische Fussnoten haben für alle Beispiele identische Bedeutung, wenn nichts anderes angegeben.

### Beispiel 1: Herstellung der Methacrylatkomponente 1

| Monomerenbeschickung: | Gewichtsteile [g] |
|---|---|
| - Methacrylsäure (MAA) | 30 |
| - Styrol (St) | 20 |
| - Methylmethacrylat (MMA) | 30 |
| - Butylmethacrylat (BuMA) | 120 |
| - Dicumylperoxid (Initiator) | 3,0 |
| - Thioglycolsäure (Kettenübertragungsmittel) | 3,5 |

| Vorlage: | |
|---|---|
| - Toluol | 60 Milliliter |
| - Dicumylperoxid (Initiator) | 3,0 g |
| - Thioglycolsäure (Kettenübertragungsmittel) | 3,5 g |

Das als Monomerenbeschickung dienende Gemisch wird mit einer 4%-igen Natriumhydroxid- und einer 20%-igen Natriumchloridlösung gewaschen und über wasserfreiem Natriumsulfat getrocknet.

Die Vorlage wird in ein Reaktionsgefäss mit mechanischem Rührer, Stickstoffspülung, Kühler, Heizung, Thermometer und einer Dosierpumpe zur kontinuierlichen Zugabe der Monomerenbeschickung gegeben. Unter Stickstoffspülung wird die Temperatur der Vorlage auf 110 °C erhöht, so dass das Toluol unter Rückfluss siedet. Dann wird die Monomerenbeschickung während eines Zeitraums von 3 Stunden zur Vorlage in dem Reaktionsgefäss gegeben, wobei gegen Ende der Zugabe die Viskosität der Mischung im Reaktionsgefäss stark zunimmt. Nach weiterem etwa sechstündigem Erhitzen der Mischung auf 110 °C wird auf Raumtemperatur abgekühlt. Das Toluol wird verdampft, das zurückbleibende Copolymer in Ether gelöst, mit Hexan gefällt, abfiltriert und getrocknet.

Das erhaltene Copolymer hat ein Molekulargewicht (M_{w}) von 10330 (gemessen mit GPC unter Verwendung eines Polystyrolstandards; M_{w}/Mₙ = 4,7), eine Glassübergangstemperatur (T_{g}-Wert) von 45,8 °C und weist 1,98 Äquivalente freie Carboxylgruppen pro Kilogramm Copolymer auf.

### Beispiel 2: Herstellung der Methacrylatkomponente 2

Es wird wie unter Beispiel 1 angegeben gearbeitet, jedoch enthält die Vorlage anstatt Toluol ein Lösungsmittelgemisch aus 70% 1-Methoxy-2-propanol, 20% 1-Methoxy-2-propylacetat und 10% Methylisobutylketon (LMGXI-Gemisch). Dieses Lösungsmittelgemisch wird weiterhin nicht wie das Toluol in Beispiel 1 entfernt, sondern es wird die nach Abkühlung des Reaktionsgemisches erhaltene Lösung des Copolymers mit Hilfe des oben genannten Lösungsmittelgemisches auf einen Feststoffgehalt von 54,6% eingestellt. Diese Lösung weist dann eine Viskosität von 460 mPa-s bei 25 °C auf.

Das hierbei erhaltene Copolymer hat ein Molekulargewicht (M_{w}) von 4300 (gemessen mit GPC unter Verwendung eines Polystyrolstandards; M_{w}/Mₙ = 3,0), eine Glassübergangstemperatur (T_{g}-Wert) von 22 °C und weist 1,80 Äquivalente freie Carboxylgruppen pro Kilogramm Copolymer auf.

### Beispiel 3: Herstellung der Methacrylatkomponente 3

| Monomerenbeschickung: | Gewichtsteile [g] |
|---|---|
| - Hydroxyethylmethacrylat | 30 |
| - Styrol (St) | 10 |
| - Methylmethacrylat (MMA) | 5,0 |
| - Butylmethacrylat (BuMA) | 60 |
| - (3-Trimethoxysilyl)propyl-methacrylat | 10,8 |
| - Dicumylperoxid (Initiator) | 2,7 |
| - Thioglycolsäure (Ketten-übertragungsmittel) | 3,2 |

| Vorlage: | |
|---|---|
| - LMGXI-Gemisch | 60 Milliliter |

| Beschickung 2: | |
|---|---|
| - LMGXI-Gemisch | 40 Milliliter |
| - Dicumylperoxid (Initiator) | 0,3 g |
| - Thioglycolsäure (Ketten-übertragungsmittel) | 0,3 g |

Die Vorlage wird in ein Reaktionsgefäss wie in Beispiel 1 verwendet gegeben und unter Stickstoffspülung zum Rückfluss erhitzt. Dann wird die Monomerenbeschickung zugegeben und weitere 3 Stunden unter Rückfluss erhitzt. Nach Zugabe der Beschickung 2 wird weitere 1,25 Stunden unter Rückfluss erhitzt. Die erhaltene Polymerlösung hat einen Feststoffgehalt von 47,4 %, das Copolymer ein Molekulargewicht (M_{w}) von 3407 (gemessen mit GPC unter Verwendung eines Polystyrolstandards; M_{w}/Mₙ = 3,3).

63,3 g der erhaltenen Polymerlösung und 0,361 g Tripropylamin werden in ein Reaktionsgefäss gegeben und unter Stickstoffatmosphäre auf 115 °C erhitzt. Dann werden 6,106 g cis-Hexahydrophthalsäureanhydrid zugegeben und 70 Minuten weiter erhitzt. Die erhaltene klare Lösung des freie Carboxylgruppen enthaltenden Acrylatpolymers (Methacrylkomponente 3) hat einen Feststoffgehalt von 23,3 % und eine Viskosität von etwa 25 mPa·s. Das Molekulargewicht des Polymers (M_{w}) beträgt 3917, seine Carboxylfunktionalität 0,32 Aeq/kg.

### Beispiel 4: Herstellung eines Halbesters aus einem Polycaprolactonpolyol und Hexahydrophthalsäureanhydrid.

59,97 g (0,39 Mol) Hexahydrophthalsäureanhydrid werden zusammen mit 1,6 g Tetramethylammoniumchlorid in ein Reaktionsgefäss mit mechanischem Rührer, Stickstoffspülung und Thermometer gegeben und unter Stickstoff auf 135 °C erhitzt. Über einen Zeitraum von 2 Stunden werden tropfenweise 100 g (0,39 Äquivalente Hydroxylgruppen) CAPA® 316 (Polycaprolactonpolyol der Firma Interox Chemicals Ltd. mit einem Molekulargewicht von 1000 und 3,89 Äquivalenten Hydroxylgruppen pro Kilogramm Substanz) zugegeben. Nach Abkühlen erhält man als Reaktionsprodukt eine viskose Flüssigkeit mit einer Viskosität von 40500 mPa·s (bei 67 °C), einem T_{g}-Wert von -19 °C und 2,41 Äquivalenten freie Carboxylgruppen pro Kilogramm Substanz.

### Beispiel 5: Herstellung eines Halbesters aus einem Polyesterpolyol und Bernsteinsäureanhydrid.

100 g K-Flex® 148 (Polyesterpolyol der Firma King Industries Inc. auf Basis linearer aliphatischer Polycarbonsäuren und Polyolen mit einer Viskosität von 4000 mPa·s bei 25 °C und 4,16 Äquivalenten freie Hydroxylgruppen pro Kilogramm Substanz) werden zusammen mit 42,2 g Bernsteinsäureanhydrid und 1,4 g Tripropylamin in ein Reaktionsgefäss mit mechanischem Rührer, Stickstoffspülung und Thermometer gegeben und auf 135 °C erhitzt. Die erhaltene Flüssigkeit einer Viskosität von 7800 mPa·s (bei 67 °C) weist einen T_{g}-Wert -27 °C und 2,94 Äquivalente freie Carboxylgruppen auf und wird nicht weiter gereinigt.

### Beispiel 6:

Eine Klarlacklösung wird durch Vermischen folgender Bestandteile bei geringer Scherkraft hergestellt:

| Bestandteile | Menge [g] |
|---|---|
| Methacrylatkomponente aus Beispiel 1 | 181,5 |
| Halbester aus Beispiel 4 | 60,0 |
| Hexahydrophthalsäurediglycidylester (6,6 Aeq/kg) | 76,5 |
| Verlaufsmittel¹⁾ | 0,75 |
| Katalysator N-Benzyldimethylamin | 3,0 |
| Lösungsmittel²⁾ | 271,5 |

| | |
|---|---|
| ¹⁾Lo 50® , ein Siliconöl der Firma Wacker Chemie, 10%-ig in Toluol | |
| ²⁾Gemisch aus 70% 1-Methoxy-2-propanol, 20% 1-Methoxy-2-propylacetat und 10% Methylisobutylketon. | |

Der Festkörpergehalt der Lacklösung beträgt 53 ± 1 Gewichtsprozent, die Viskosität bei 25 °C 150 mPa·s (ICI CONE & PLATE VISCOSIMETER). Der Festkörpergehalt für eine Spritzviskosität entsprechend 18 Sekunden Auslaufzeit (DIN-4-Becher) beträgt 47 Gewichtsprozent. Der Klarlack wird auf ein Aluminiumsubstrat und ein Floridasubstrat (Polyester/Melaminlaminat) aufgebracht. Nach einer Härtung von 30 Minuten bei 140 °C weist die Beschichtung eine Schichtdicke von 25 µm mit folgenden Eigenschaften auf:

| | Aluminium | Florida |
|---|---|---|
| Erichsentiefziehtest [mm] | 9,9 | 5,8⁴⁾ |
| Schlagverformung [kg·cm] D/R³⁾ | 160/100 | 80/80⁴⁾ |
| Härte nach Persoz [s] | 252 | 258 |
| Acetontest⁵⁾ | 3/3 | 3/3 |
| Klebetest⁶⁾ | Gt0 | Gt0 |

| | | |
|---|---|---|
| ³⁾ Die Schlagverformung wird bestimmt, indem man einen Stempel bekannter Masse aus bestimmter Höhe auf das beschichtete Substrat fallen lässt. Der angegebene Wert ist das Produkt aus der Masse des Stempels in kg und der grössten Höhe in cm, bei der die Beschichtung noch unbeschädigt bleibt, d.h. unter 10-facher Vergrösserung keine Risse in der Beschichtung festgestellt werden können. Der Zusatz "R" bedeutet, dass der Stempel auf die der Beschichtung abgewandte Seite des Substrats auftrifft, ein Zusatz "D", dass er auf die beschichtete Seite auftrifft. | | |
| ⁴⁾ Substrat bricht an diesem Punkt | | |
| ⁵⁾ Erster Wert: Der Acetontest wird als Reibtest folgendermassen durchgeführt: Ein Wattebausch wird mit Aceton getränkt und 20 mal über eine Stelle der beschichteten Fläche hin und her gerieben. Das Ergebnis wird gemäss der folgenden fünfteiligen Skala (DIN 53320) beurteilt: 0 = unverändert; 1 = bremsend, nicht mit dem Fingernagel kratzbar; 2 = schwer kratzbar, evtl. Watte gefärbt; 3 = erweicht, leicht kratzbar; 4 = beginnende Ab- oder Auflösung; 5 = vollständige Auflösung. Zweiter Wert gemäss der gleichen Skala aber nach einminütigem Eintauchen in Aceton. | | |
| ⁶⁾ Gitterschnitttest nach DIN 53151 : Mit Hilfe genau definierter Schneidegeräte wird ein quadratisches Gittermuster in die Beschichtung geschnitten, wobei der Abstand der einzelnen Gitterlinien je nach Dicke der Beschichtung 1, 2 oder 3 Millimeter betragen muss. Als Mass für die Beurteilung der Haftfestigkeit wird der Prozentsatz der hierbei abplatzenden Teilquadrate der Gitterfläche abgeschätzt: Gt0 = 0%; Gt1 = ca. 5%; Gt2 = ca. 15%; Gt 3 = ca. 35%; Gt4 = 65% und mehr; Gt 5 = 100%. | | |

### Beispiel 7:

Es wird wie unter Beispiel 6 verfahren, jedoch die folgende Lackzusammensetzung verwendet:

| Bestandteile | Menge [g] |
|---|---|
| Methacrylatkomponente aus Beispiel 2 | 186,2 |
| Halbester aus Beispiel 4 | 60,0 |
| Hexahydrophthalsäurediglycidylester (6,6 Aeq/kg) | 72,0 |
| Verlaufsmittel¹⁾ | 0,75 |
| UV-Absorber CGL 400 (2%) | 6,4 |
| Antioxidationmittel Tinuvin® 123 (1%) | 3,2 |
| 1,8-Diazabicyclo[5,4,0]-7-undecen als Katalysator | 5,8 |
| Lösungsmittel²⁾ | 267,0 |

Der erhaltene Lack weist einen Festkörpergehalt von 55 Gewichtsprozent auf. Er wird in einer Dicke von 35 µm auf ein Aluminium- und ein Floridasubstrat aufgebracht, die mit einem kommerziellen Automobilmetalliclack (VWL 97 A /Firma BASF) beschichtet sind.

Die 35 µm starke Beschichtung weist nach 30-minütiger Härtung bei 130 °C folgende Eigenschaften auf:

| | Aluminium | Florida |
|---|---|---|
| Erichsentiefziehtest [mm] | 9,6 | 6,1 ⁴⁾ |
| Schlagverformung [kg·cm] D/R³⁾ | 160/160 | 80/80⁴⁾ |
| Härte nach Persoz [s] | 197 | 173 |
| Acetontest⁵⁾ | 3/3 | 3/3 |
| Klebetest⁶⁾ | 0 | 0 |
| Yi¹⁰⁾/Glanz (20°)⁹⁾ | -0,9/94 | -2/82 |
| Yi/Glanz (20°) | | |
| nach 500h Bewitterung⁷⁾ | -1,6/89 | -2/86 |
| nach 1900h Bewitterung | -2/84 | -2/85 |
| nach 4400h Bewitterung | 0/54 | -1/54 |

| | | |
|---|---|---|
| ⁷⁾ Schnellbewitterung in einem Weatherometer ATLAS CI 65, Witterungscyclus NBR 180 | | |
| ⁹⁾ Messung des Glanzes unter einem Winkel von 20° | | |
| ¹⁰⁾ Vergilbung (Yellowness), bestimmt mit Spectralphotometer nach DIN 6172 | | |

### Beispiel 8:

Es wird ein pigmentierter Basislack mit der nachfolgenden Zusammensetzung hergestellt:

| Bestandteile | Menge [g] |
|---|---|
| Methacrylatkomponente aus Beispiel 1 | 136,0 |
| Halbester aus Beispiel 3 | 64,0 |
| Hexahydrophthalsäurediglycidylester (6,6 Aeq/kg) | 64,2 |
| Titandioxid CRONOS® 2160 | 113,2 |
| Verlaufsmittel¹⁾ | 0,75 |
| Katalysator N-Benzyldimethylamin | 2,65 |
| Lösungsmittel²⁾ | 151,0 |

Der Lack hat einen Feststoffgehalt von 71 Gewichtsprozent und eine Viskosität von 600 mPa·s. Die Formulierung wird 30 µm dick auf ein Aluminiumsubstrat aufgetragen und bei den in der nachfolgenden Tabelle angegebenen Temperaturen jeweils 30 Minuten lang gehärtet. Folgende Eigenschaften werden für die Beschichtung beobachtet:

| | Härtung bei | | |
|---|---|---|---|
| | 100 °C | 120 °C | 140 °C |
| Erichsentiefziehtest [mm] | 9,8 | 9,6 | 9,4 |
| Schlagverformung [kg·cm] R³⁾ | <20 | 160 | 160 |
| Härte nach Persoz [s] | 76 | 176 | 197 |
| Acetontest⁵⁾ | 3/4 | 3/3 | 3/3 |
| Klebetest⁶⁾ | Gt0 | Gt0 | Gt0 |
| Yi/Glanz (60°)⁸⁾ | - | -3,3/89 | -3,1/87 |
| Yi/Glanz (60°) | | | |
| nach 2000h Bewitterung⁷⁾ | - | -2,5/85 | -2,5/86 |
| nach 3300h Bewitterung | - | -2/52 | -3/53 |

| | | | |
|---|---|---|---|
| ⁸⁾ Messung des Glanzes unter einem Winkel von 60° | | | |

Besonders bemerkenswert ist, dass der Glanz trotz Abwesenheit eines UV-Absorbers und eines Antioxidationsmittels auch nach 2000-stündiger Bewitterung nur sehr wenig abnimmt.

### Beispiel 9:

Es wird ein Lack folgender Zusammensetzung hergestellt:

| Bestandteile | Menge [g] |
|---|---|
| Methacrylatkomponente aus Beispiel 3 | 150 |
| Halbester aus Beispiel 4 | 50,0 |
| Hexahydrophthalsäurediglycidylester (6,6 Aeq/kg) | 51,3 |
| Byk 300 | 1,4 |
| UV-Absorber CGL 400 (2%) | 5,0 |
| Antioxidationmittel Tinuvin® 123 (1%) | 2,5 |
| 1,8-Diazabicyclo[5,4,0]-7-undecen als Katalysator | 7,5 |
| Lösungsmittel²⁾ | 493,7 |

Der erhaltene Lack weist einen Festkörpergehalt von 35 Gewichtsprozent und eine Viskosität von 40 mPa·s auf. Er wird auf ein Aluminium- und ein Floridasubstrat aufgebracht, die mit einem kommerziellen Automobilmetalliclack (VWL 97 A der Firma BASF) beschichtet sind.

Die Beschichtung weist nach 30-minütiger Härtung bei 130 °C folgende Eigenschaften auf:

| | Aluminium | Florida |
|---|---|---|
| Schichtdicke [µm] | 25-35 | 35-40 |
| Erichsentiefziehtest [mm] | 10,3 | 6,0⁴⁾ |
| Schlagverformung [kg·cm] D/R³⁾ | 160/80 | 80/80⁴⁾ |
| Härte nach Persoz [s] | 136 | 134 |
| Acetontest⁵⁾ | 3/3 | 3/3 |
| Klebetest⁶⁾ | Gt1 | Gt0 |
| Yi¹⁰⁾/Glanz (20°) | - | 0.3/84 |
| Yi/Glanz (20°) | | |
| nach 800h Bewitterung⁷⁾ | - | -2/85 |
| nach 2400h Bewitterung | - | -2/54 |

### Beispiel 10: Herstellung von 1,2,4-Cyclohexyltricarbonsäuretriglycidylester.

Für die Glycidylisierung von 1,2,4-Cyclohexyltricarbonsäure (erhältlich z. B. durch Hydrierung von Trimellitsäure gemäss der US-A-3,444,237) wird eine Apparatur eingesetzt, die es gestattet, Epichlorhydrin als Schleppmittel für die Wasserabscheidung unter vermindertem Druck einzusetzen. Diese Apparatur besteht aus einem von aussen heizbaren 250 ml-Reaktiongefäss mit Rührer, Thermometer, einem gut wirkenden Intensivkühler und zwei Tropftrichtern mit Druckausgleich. Angeschlossen sind weiterhin eine Wasserstahlpumpe und eine Wasserabscheider, der die Rückführung von unverbrauchtem Epichlorhydrin in das Reaktionsgefäss ermöglicht.

20 g 1,2,4-Cyclohexyltricarbonsäure, 152 ml Epichlorhydrin und 1,6 g einer 50%-igen wässerigen Tetramethylammoniumchloridlösung werden auf ca. 80 °C erhitzt. Unter guter Rührung wird vorsichtig Vakuum an die Apparatur angelegt, bis das Epichlorhydrin heftig am Rückfluss siedet. Der pH-Wert der Reaktionsmischung wird ständig überwacht und das Gemisch weiter reagieren lassen, bis es basisch wird. Dann wird auf 50 °C abgekühlt und ein Druck von 0,11 bar (80 mm Hg) eingestellt. Innerhalb von 3 Stunden werden aus dem zweiten Tropftrichter 24,28 g einer 50%-igen Natriumhydroxidlösung zugetropft, wobei die Temperatur bei ca. 46 bis 50 °C gehalten wird. Das eingebrachte und gebildete Wasser destillieren zusammen mit Epichlorhydrin als azeotropes Gemisch ab. Das in dem Wasserabscheider vom Wasser abgetrennte Epichlorhydrin wird kontinuierlich in das Reaktionsgemisch zurückgeführt. Nach dem Zutropfen aller Natronlauge wird noch eine Stunde bei ca. 46°C nachreagieren lassen. Insgesamt werden etwa 15 g Wasser entfernt. Die erhaltene Suspension wird danach bei Normaldruck über Kieselgur als Filtrierhilfsmittel filtriert. Die im Reaktionsgefäss verbleibenden Reste des Reaktionsgemisches werden mit Ethylacetat aufgenommen und ebenfalls filtriert, wonach der Filterkuchen mit Ethylacetat gewaschen wird. Die vereinten Filtrate werden mit 10%-iger Natriumhydrogenphosphatlösung extrahiert und das extrahierte Gemisch bei 80 °C unter Vakuum am Rotationsverdampfer eingeengt. Restspuren des Lösungsmittels werden danach während 20 Minuten bei 120°C und unter Hochvakuum entfernt.

Das gewünschte Produkt wird als viskose Flüssigkeit (4750 mPa·s bei 25 °C) erhalten und weist einen Epoxidgehalt von 7,44 Aeq/kg Substanz auf. Sein Molekulargewicht (M_{w}) wird mit GPC zu 335 (M_{w}/Mₙ = 1,093) bestimmt.

### Beispiel 11:

Es wird ein Klarlack folgender Zusammensetzung hergestellt:

| Bestandteile | Menge [g] |
|---|---|
| Methacrylatkomponente aus Beispiel 2 | 153,9 |
| Halbester aus Beispiel 4 | 102,6 |
| Epoxidharz aus Beispiel 10 | 47 |
| Verlaufsmittel¹⁾ | 1,1 |
| UV-Absorber CGL 400 (2%) | 6,1 |
| Antioxidationmittel Tinuvin® 123 (1%) | 3,0 |
| 1,8-Diazabicyclo[5,4,0]-7-undecen | 5,3 |
| Lösungsmittel²⁾ | 206,1 |

Der erhaltene Lack weist einen Festkörpergehalt von 59,5 Gewichtsprozent und eine Viskosität von 600 mPa·s auf. Der Klarlack wird jeweils auf ein Aluminiumsubstrat sowie auf ein Floridasubstrat, das mit dem Automobilmetalliclack VWL 97 A der Firma BASF beschichtet ist, aufgebracht. Nach einer Härtung von 30 Minuten bei 130 °C weisen die Beschichtungen folgende Eigenschaften auf (Schichtdicke 25 µm):

| Substrat | Aluminium | Florida lackiert |
|---|---|---|
| Erichsentiefziehtest [mm] | 10,3 | 4,8⁴⁾ |
| Schlagverformung [kg·cm] D/R³⁾ | 160/160 | 80/80 |
| Härte nach Persoz [s] | 197 | 171 |
| Acetontest⁵⁾ | 3/3 | 3/3 |
| Klebetest⁶⁾ | Gt0 | Gt0 |
| Yi/Glanz (20°) | - | -0,3/85 |
| Yi/Glanz (20°) | | |
| nach 2000h Bewitterung⁷⁾ | - | -1/58 |

### Beispiel 12:

Es wird ein Klarlack folgender Zusammensetzung hergestellt:

| Bestandteile | Menge [g] |
|---|---|
| Kommerzielle Methacrylatkomponente Joncryl® SCX817C (0,982 AeqCOOH/kg) | 280 |
| Halbester aus Beispiel 4 | 120 |
| Hexahydrophthalsäurediglycidylester (6,6 Aeq/kg) | 81,7 |
| Verlaufsmittel (Byk® 300) | 1,6 |
| UV-Absorber CGL 400 (2%) | 9,6 |
| Antioxidationmittel Tinuvin® 123 (1%) | 4,8 |
| 1,8-Diazabicyclo[5,4,0]-7-undecen | 8,4 |
| Lösungsmittel²⁾ | 336 |

Der erhaltene Lack weist einen Festkörpergehalt von 60,1 Gewichtsprozent und eine Viskosität von 1160 mPa·s auf. Der Klarlack wird jeweils auf ein Aluminiumsubstrat sowie auf ein Floridasubstrat, das mit dem Automobilmetalliclack VWL 97 A der Firma BASF beschichtet ist, aufgebracht. Nach einer Härtung von 30 Minuten bei 130 °C weisen die Beschichtungen folgende Eigenschaften auf (Schichtdicke 30 µm):

| Substrat | Aluminium | Florida lackiert |
|---|---|---|
| Erichsentiefziehtest [mm] | 10,3 | 5,4⁴⁾ |
| Schlagverformung [kg·cm] D/R³⁾ | 160/100 | 80/80 |
| Härte nach Persoz [s] | 252 | 240 |
| Acetontest⁵⁾ | 3/3 | 3/3 |
| Klebetest⁶⁾ | Gt0 | Gt0 |
| Yi/Glanz (20°) | - | -1/87 |
| Yi/Glanz (20°) | | |
| nach 2600h Bewitterung⁷⁾ | - | -1/80 |

### Beispiel 13:

Es wird ein Klarlack folgender Zusammensetzung hergestellt:

| Bestandteile | Menge [g] |
|---|---|
| Kommerzielle Methacrylatkomponente Joncryl® SCX817C (0,982 AeqCOOH/kg) | 280 |
| Halbester aus Beispiel 4 | 120 |
| Gemisch aus 70 Gew.-T. Hexahydrophthalsäurediglycidylester/ 30 Gew.-T. Trimellitsäuretriglycidylester (6,86 Aeq/kg) | 85,2 |
| Verlaufsmittel (Byk® 300) | 1,0 |
| UV-Absorber CGL 400 (2%) | 9,6 |
| Antioxidationmittel Tinuvin® 123 (1%) | 4,8 |
| 1,8-Diazabicyclo[5,4,0]-7-undecen | 8,2 |
| Lösungsmittel²⁾ | 335,4 |

Der erhaltene Lack weist einen Festkörpergehalt von 60,3 Gewichtsprozent und eine Viskosität von 1120 mPa·s auf. Der Klarlack wird jeweils auf ein Aluminiumsubstrat sowie auf ein Floridasubstrat, das mit dem Automobilmetalliclack VWL 97 A der Firma BASF beschichtet ist, aufgebracht. Nach einer Härtung von 30 Minuten bei 130 °C weisen die Beschichtungen folgende Eigenschaften auf (Schichtdicke 40 µm):

| Substrat | Aluminium | Florida lackiert |
|---|---|---|
| Erichsentiefziehtest [mm] | 10,3 | 5,7⁴⁾ |
| Schlagverformung [kg·cm] D/R³⁾ | - | 80/60-70 |
| Härte nach Persoz [s] | 289 | 266 |
| Acetontest⁵⁾ | 3/0-1 | 3/0-1 |
| Klebetest⁶⁾ | Gt0 | Gtl |
| Yi/Glanz (20°) | - | -1/85 |
| Yi/Glanz (20°) | | |
| nach 2000h Bewitterung⁷⁾ | - | -1/80 |

## Patentansprüche

1. Härtbare Zusammensetzung enthaltend
A) ein Gemisch aus
A1) mindestens einem Polymer auf Basis von Acrylat- und/oder Methacrylatmonomeren, das durchschnittlich 0,1 bis 4,0 Äquivalente freie Carboxylgruppen pro Kilogramm Polymer aufweist, und
A2) mindestens einem carboxylterminierten Polyester, der durchschnittlich 0,2 bis 6 Äquivalente freie Carboxylgruppen pro Kilogramm Polyester aufweist, wobei Bestandteil A1) 50 bis 90 Gewichtsprozent und Bestandteil A2) den Rest der Komponente A ausmachen;
B) einen oder mehrere Glycidylester cycloaliphatischer Carbonsäuren mit 2 oder mehr Epoxidgruppen pro Molekül oder ein Gemisch, das aus einem oder mehreren Glycidylestern cycloaliphatischer Carbonsäuren mit 2 oder mehr Epoxidgruppen pro Molekül und einem oder mehreren Glycidylestern aromatischer Carbonsäuren mit 2 oder mehr Epoxidgruppen pro Molekül besteht;
C) einen Katalysator zur Beschleunigung der thermischen Reaktion von Epoxid- und Carboxylgruppen und
D) ein inertes Lösungsmittel,
wobei das molare Verhältnis von freien Carboxylgruppen zu Epoxidgruppen in der Zusammensetzung 0,3 bis 3 beträgt und die Zusammensetzung ausser der Komponente B keine Bestandteile mit Epoxidgruppen aufweist.

2. Zusammensetzung nach Anspruch 1, die cis-Hexahydrophthalsäurediglycidylester und/oder 1,2,4-Cyclohexantricarbonsäuretriglycidylester enthält.

3. Zusammensetzung nach Anspruch 1, die als Komponente (B) einen oder mehrere Glycidylester cycloaliphatischer Carbonsäuren enthält.

4. Zusammensetzung nach Anspruch 1, die als Komponente (B) ein Gemisch enthält, das aus einem oder mehreren Glycidylestern cycloaliphatischer Carbonsäuren mit 2 oder mehr Epoxidgruppen pro Molekül und einem oder mehreren Glycidylestern aromatischer Carbonsäuren mit 2 oder mehr Epoxidgruppen pro Molekül besteht.

5. Zusammensetzung nach Anspruch 1 bis 4, die ein Epoxidharz mit 3 oder mehr Epoxidgruppen pro Molekül enthält.

6. Zusammensetzung nach Anspruch 1 bis 5, bei der die Komponente A2) ein Halbester der Formel 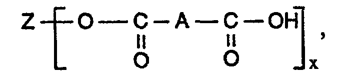 ist, worin
Z den Rest eines Polyester- oder Polylactonpolyols der Formel Z(OH)ₓ ohne seine terminalen Hydroxylgruppen darstellt,
A den Rest einer von ihren 1,2-ständigen Carboxylgruppen befreiten 1,2-Carbonsäure der Formel A(COOH)₂ symbolisiert, und
x eine ganze Zahl von mindestens 2 ist, und
wobei die Halbester ein Molekulargewicht von 500 bis 5000 und eine Glasübergangstemperatur unter 30 °C aufweisen.

7. Zusammensetzung nach Anspruch 6, wobei A den Rest einer cycloaliphatischen Carbonsäure darstellt.

8. Zusammensetzung nach Anspruch 6 oder 7, wobei die Komponente A2) ein Halbester eines Polylactonpolyols ist, das eine Struktur der Formel 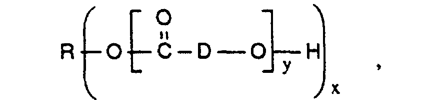 aufweist, worin
R dem Rest eines x-wertigen Initiatorpolyols R(OH)ₓ ohne seine Hydroxylgruppen darstellt,
x die in Anspruch 7 angegebenen Bedeutung hat,
y eins oder eine ganze Zahl grösser als 1 bedeutet und
D einer Alkylengruppe der Struktur 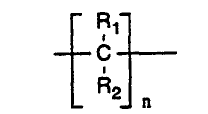 mit insgesamt maximal 12 Kohlenstoffatomen entspricht, wobei
R₁ und R₂ unabhängig voneinander ein Wasserstoffatom oder einen C₁-C₃-Alkylrest darstellen und
n eine ganze Zahl von 5 bis 8 ist.

9. Zusammensetzung nach Anspruch 8, wobei
n gleich 5 ist und
R₁ und R₂ beide ein Wasserstoffatom darstellen.

10. Zusammensetzung nach Anspruch 8 oder 9, wobei
x gleich 2 oder 3 ist.

11. Cyclohexanpolycarbonsäureglycidylester mit mehr als 2 Glycidylestergruppen.

12. 1,2,4-Cyclohexyltricarbonsäuretriglycidylester gemäss Anspruch 11.

13. Gemisch bestehend aus einem oder mehreren Cyclohexanpolycarbonsäureglycidylestern gemäss Anspruch 11 und einem oder mehreren Glycidylestern aromatischer Carbonsäuren.

14. Verfahren zur Lackierung von Gegenständen, insbesondere von Automobilteilen, bei dem der Gegenstand mit einer Zusammensetzung nach Anspruch 1 bis 10 beschichtet wird und die Beschichtung getrocknet und bei einer Temperatur von 60 bis 180 °C, vorzugsweise von 80 bis 130 °C, gehärtet wird.

## Claims

1. A curable composition comprising
A) a mixture of
A1) at least one polymer based on acrylate and/or methacrylate monomers and containing on average 0.1 to 4.0 equivalents of free carboxyl groups per kilogram of polymer, and
A2) at least one carboxyl-terminated polyester which contains on average 0.2 to 6 equivalents of free carboxyl groups per kilogram of polyester such that component A1) constitutes 50 to 90 per cent by weight, and component A2) makes up the remainder, of the mixture A;
B) one or more glycidyl esters of cycloaliphatic carboxylic acids containing 2 or more epoxy groups per molecule, or a mixture of one or more glycidal esters of cycloaliphatic carboxylic acids, and one or more
glycidyl esters of aromatic carboxylic acids containing 2 or more epoxy groups per molecule; C)a catalyst for speeding up the thermal reaction of epoxy and carboxyl groups; and
D)an inert solvent;
the molar ratio of free carboxyl groups to epoxy groups in said composition being 0.3 to 3, and said composition containing, except for component B, no components carrying epoxy groups.

2. A composition according to claim 1, comprising cis-hexahydrophthalic acid diglycidyl ester and/or 1,2,4-cyclohexanetricarboxylic acid triglycidyl ester.

3. A composition according to claim 1, comprising as component (B) one or more glycidyl esters of cycloaliphatic carboxylic acids.

4. A composition according to claim 1, comprising as component (B) a mixture of one or more glycidyl esters of cycloaliphatic carboxylic acids containing 2 or more epoxy groups per molecule and one or more glycidyl esters of aromatic carboxylic acids containing 2 or more epoxy groups per molecule.

5. A composition according to claims 1 to 4, comprising an epoxy resin containing 3 or more epoxy groups per molecule.

6. A composition according to claims 1 to 5, wherein component A2) is a half-ester of formula 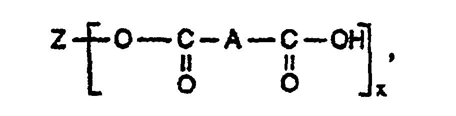 in which
Z is the radical of a polyester polyol or polylactone polyol of formula Z(OH)ₓ without its hydroxyl end groups,
A is the radical of a 1,2-carboxylic acid of formula A(COOH)₂ without its carboxyl groups in 1,2-position, and
x is an integer of at least 2,
which half-ester has a molecular weight of 500 to 5000 and a glass transition temperature below 30°C.

7. A composition according to claim 6, wherein A is the radical of a cycloaliphatic carboxylic acid.

8. A compositing according to claim 6 or 7, wherein component A2) is a half-ester of a polylactone polyol having a structure of formula 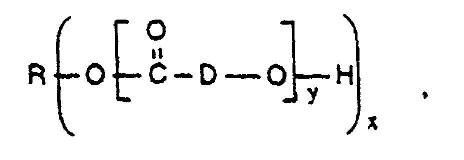
in which R is the radical of an initiator polyol R(OH)ₓ of valency x without its hydroxyl groups x is a defined in claim 7,
y is one or an integer greater than 1, and
D corresponds to an alkylene group of structure 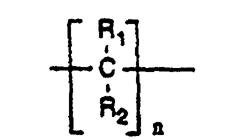 containing a maximum total number of 12 carbon atoms, in which R₁ and R₂ are each independently of the other a hydrogen atom or a C₁-C₃alkyl radical, and
n is an integer from 5 to 8.

9. A composition according to claim 8, wherein n is 5 and R₁ and R₂ are both a hydrogen atom.

10. A composition according to claim 8 or 9, wherein x is 2 or 3.

11. A cyclohexanepolycarboxylic acid glycidyl ester containing more than 2 glycidyl ester groups.

12. 1,2,4-Cyclohexyltricarboxylic acid triglycidyl ester, according to claim 11.

13. A mixture consisting of one or more cyclohexanepolycarboxylic acid glycidyl esters according to claim 11 and one or more glycidyl esters of aromatic carboxylic acids.

14. A method of coating objects, preferably automobile parts, which comprises coating the object with a composition according to claims 1 to 10, drying the coating and effecting a cure at a temperature from 60 to 180°C, preferably from 80 to 130°C.

## Revendications

1. Composition durcissable contenant
A) un mélange de
A1) au moins un polymère à base de monomères acrylate et/ou méthacrylate qui présente en moyenne de 0,1 à 4,0 équivalents de groupes carboxyle libres par kilogramme de polymère, et
A2) au moins un polyester à groupes terminaux carboxyle, qui présente de 0,2 à 6 équivalents de groupes carboxyle libres par kilogramme de polyester, le constituant A1) représente de 50 à 90 % en masse et le constituant A2) complète le reste du composant A) ;
B) un ou plusieurs esters glycidyliques d'acides carboxyliques cycloaliphatiques ayant 2 ou plus de deux groupes époxyde par molécule ou un mélange qui est constitué par un ou plusieurs esters glycidyliques d'acides carboxyliques cycloaliphatiques présentant 2 ou plus de deux groupes époxyde par molécule et un ou plusieurs esters glycidyliques d'acides carboxyliques aromatiques avec 2 ou plusieur groupes époxyde par molécule ;
C) un catalyseur pour accélérer la réaction thermique de groupes époxyde et carboxyles et
D) un solvant inerte,
le rapport molaire des groupes carboxyle libres aux groupes époxyde dans la composition est de 0,3 à 3 et la composition ne présente pas, outre le composant B, de constituants avec groupes époxyde.

2. Composition selon la revendication 1, qui contient l'ester diglycidylique de l'acide cis-hexahydrophtalique et/ou l'ester triglycidylique de l'acide 1,2,4-cyclohexanetricarboxylique.

3. Composition selon la revendication 1, qui contient comme composant (B) un ou plusieurs esters glycidyliques d'acides carboxyliques cycloaliphatiques.

4. Composition selon la revendication 1, qui contient en tant que composant (B) un mélange qui est constitué par un ou plusieurs esters glycidyliques d'acides carboxyliques cycloaliphatiques ayant 2 ou plus de deux groupes époxyde par molécule et un ou plusieurs esters glycidyliques d'acides carboxyliques aromatiques ayant 2 ou plus de deux groupes époxyde par molécule.

5. Composition selon la revendication 1 à 4, qui contient une résine époxyde ayant 3 ou plus groupes époxydes par molécule.

6. Composition selon 1. revendication 1 à 5, où le composant A2) est un hémi-ester de formule 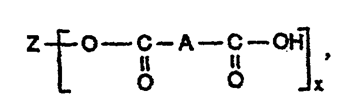 où
Z représente le reste d'un polyester-polyol ou polylactone-polyol de formule Z(OH)ₓ sans ses groupes hydroxyle terminaux,
A représente le reste d'un acide 1,2-carboxylique dépourvu de ses groupes carboxyle en position 1,2, de formule A(COOH)₂, et
x est un entier d'au moins 2, et
l'hémi-ester présente une masse molaire de 500 à 5000 et une température de transition vitreuse inférieure à 30°C.

7. Composition selon la revendication 6, où A représente le reste d'un acide carboxylique cycloaliphatique.

8. Composition selon la revendication 6 ou 7, où le composant A2) est un hémi-ester d'un polylactonepolyol qui présente une structure de formule 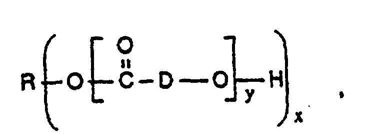 où
R représente le reste d'un polyol-amorceur R(OH)ₓ à fonctionnalité x dépourvu de ses groupes hydroxyle,
x possède l'une des significations données à la revendication 7,
y vaut 1 ou est un entier supérieur à 1, et
D représente un groupe alkylène répondant à la structure 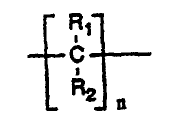 ayant au total 12 atomes de carbone.,
R₁ et R₂ représentant, indépendamment l'un de l'autre, un atome d'hydrogène ou un reste alkyle en C₁-C₃ et
n est un entier de 5 à 8.

9. Composition selon la revendication 8, où
n est égal à 5 et
R₁ et R₂ représentent, les deux, un atome d'hydrogène.

10. Compositon selon la revendication 8 ou 9, où
x est égal à 2 ou 3.

11. Ester gycidylique d'acides cyclohexanepolycarboxyliques ayant plus de deux groupes ester glycidylique.

12. Ester triglycidylique de l'acide 1,2,4-cyclohexanetricarboxylique selon la revendication 11.

13. Mélange constitué par un ou plusieurs esters glycidyliques d'acides cyclohexanepolycarboxyliques selon la revendication 11 et un ou plusieurs esters glycidyliques d'acides carboxyliques aromatiques.

14. Procédé pour le laquage d'objets, notamment de pièces d'automobile, dans lequel l'objet est revêtu d'une composition selon la revendication 1 à 10 et le revêtement est séché et durci à une température de 60 à 180°C, de préférence de 80 à 130°C.
